# EUROPEAN PATENT SPECIFICATION

(11) **EP 3 530 303 B1**
(45) Date of publication and mention of the grant of the patent: **17.07.2024**
(21) Application number: 18158167.9
(22) Date of filing: 22.02.2018
(51) Int. Cl.: A61M 5/00, B65D 81/00

(54) **STORAGE CONTAINER FOR ELECTRONIC INJECTION DEVICE**
AUFBEWAHRUNGSBEHÄLTER FÜR EINE ELEKTRONISCHE INJEKTIONSVORRICHTUNG
CONTENEUR DE STOCKAGE POUR DISPOSITIF D'INJECTION ÉLECTRONIQUE

(43) Date of publication of application: 28.08.2019
(73) Proprietor: Ares Trading S.A., 1170 Aubonne (CH)
(72) Inventor: CIMPRICH, Thomas, 1028 Préveranges (CH)
(74) Representative: Merck Serono S.A. Intellectual Property

(56) References cited:
- EP-A1- 2 573 007
- WO-A1-03/064281
- WO-A1-2017/057477
- US-A1- 2007 234 636

## Description

### BACKGROUND OF THE INVENTION

The present invention is directed to storage containers for electronic injection devices, in particular for electronic injection devices comprising multiple doses of a medication. These storage containers are for storage of such electronic injection devices when not being in use, in particular during periods in between injections according to a dosage regimen of a medication.

Patients suffering from a variety of diseases often are able to treat themselves by self-administering appropriate doses of a medication. One type of delivery device that can be used by patients or care-givers includes electronic injection devices. Multi-dose cartridges in such electronic delivery devices make the use and adherence to a particular dosing regimen more convenient to the user. The electronics in such delivery devices add to the convenience of the patient or care giver by controlling the injection through operating for example the needle injection or delivery of the medication through a needle.

In a typical operation, users will store the electronic injection device containing the medication inside a refrigerator, for example a home refrigerator. Prior to use, users/patients (or care-givers) commonly remove the device (typically containing the medication) from the refrigerator and allow it to reach ambient temperature before injection. Once delivery is completed, the device containing the remaining medication is then returned to the refrigerator. Thus, the device stored in the refrigerator when not being used, is removed for use from the refrigerator for a short time, such as for example for 30 minutes, and after use is then placed back in the refrigerator for storage until its next use.

Removing and/or returning the electronic drug delivery device from the refrigerator, particularly in regions where there is a high ambient temperature and humidity, may result in the formation of condensation on and in the device. This condensation has the potential to damage the electronics and cause the device to malfunction or become inoperable.

Electronic injection devices are often provided with a storage case in which the device can be protectively stored when not in use. Typically, the device can be placed within a complementarily shaped mold provided in an interior space. However, such cases typically do not protect against the condensation of the electronic injection device when transferred from a location of storage to the use environment for the user which two locations have different temperatures. This is particularly problematic when the user of the electronic injection device is located in a region where there is a high ambient temperature and humidity.

WO03/064281 A1 discloses a storage case for a portable medication delivery device.

Thus, it would be desirable to provide a storage case for an electronic injection device that addresses at least one of these shortcomings of the prior art storage cases.

### BRIEF SUMMARY OF THE INVENTION

The invention is as defined in independent claim 1. Preferred embodiments are as defined in dependent claims 2-13.

In one embodiment, the present invention provides a storage container for an electronic injection device comprising, a bottom part having an outer surface and defining an interior space for holding the electronic injection device, a movable top part for closing the interior space of the bottom part forming when closed a hermetically sealed interior compartment, and a desiccant in air flow communication with the interior compartment, wherein the desiccant can be replaceable and/or rechargeable. The desiccant can be in a removable compartment in the interior space. Continued use of the storage container after the desiccant has been saturated with moisture can be through replacement of the desiccant compartment. Further, the used desiccant compartment can be recharged for example by evaporating the moisture captured by the desiccant in the desiccant compartment.

The top part or lid part may be removable from the bottom part to open and close the storage container. Alternatively, both parts are connected by one or more hinges so as to provide a pivotal means for opening and closing the storage container.

In an alternative embodiment of the present invention, the outer surface of the bottom part comprises a material for creating a humidity barrier and which material is of a thickness that allows an equilibration period for equilibrating the temperature inside the interior compartment and outside of the storage container of less than 60 min.

In an alternative embodiment the bottom part of the storage container comprises feet on which the bottom part rests when placed in upright position when used to store an electronic injection device. The feet can provide airflow and ventilation underneath the storage container for increased heat exchange between the interior space and the ambient environment.

An advantage of the present invention is that a storage container for an electronic injection device is provided which reduces the likelihood of condensation forming on the device when not in use, which condensation could adversely impact the reliability of the device. Another advantage of the present invention is that a storage container is provided which while protecting the electronic injection device from potentially damaging humidity can equilibrate sufficiently rapidly to room temperature after removal from a cold storage compartment allowing the user or care-giver to manage the injection of the medication.

### BRIEF DESCRIPTION OF THE DRAWINGS

The above-mentioned and other advantages and objects of this invention will become more apparent, and the invention itself will be better understood by reference to the following description of an embodiment of the invention together with the accompanying drawings, wherein:

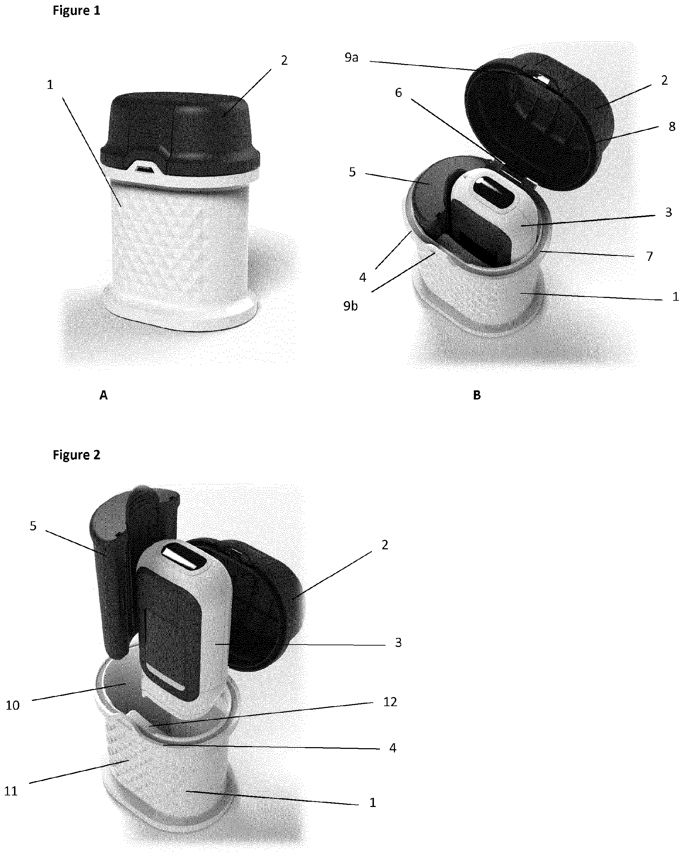

FIG. 1 is a representation of a storage container for an electronic injection device. In figure 1A the container is in a closed view providing a seal between the bottom part and the top part. In figure 1 B the storage container is opened at the hinge between the bottom part and top part showing the electronic injection device and a compartment for desiccant placed in the bottom part of the storage container.
FIG. 2 is a top view of the storage container showing the interior space, for placing the electronic injection device and the compartment for desiccant. The outside surface of the bottom part is rough-patterned. Also shown is a mold inside the interior space to hold the electronic injection device in place.

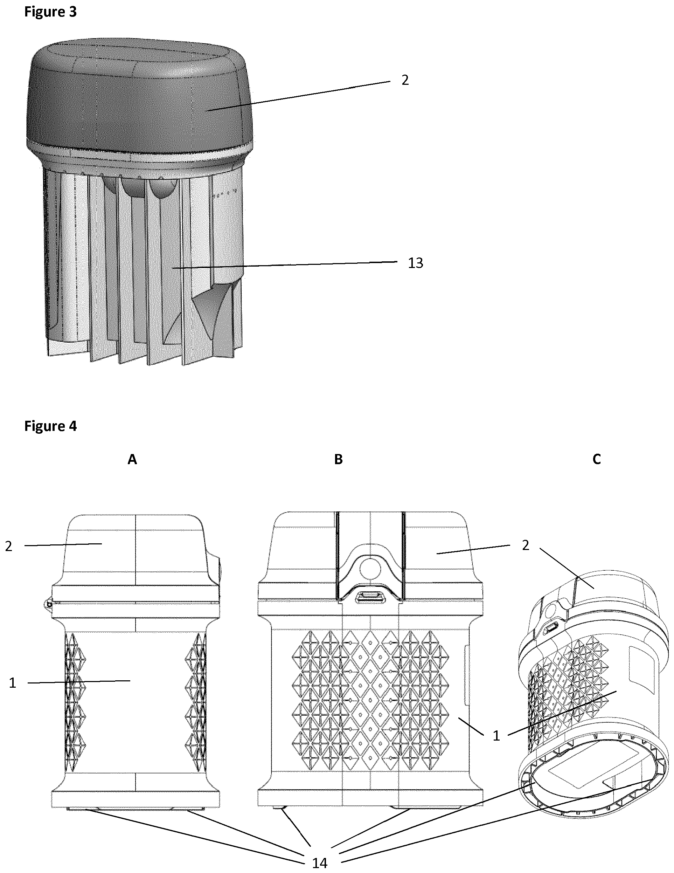

FIG. 3 is a representation of the storage container in a closed view from which the outside surface of the bottom part has been removed revealing an interior lining of the interior space of the bottom part of the storage container.
FIG. 4 is a representation of the storage container showing the presence of feet supporting the bottom part of the storage container. Figures 4A, 4B and 4C provide different views showing the feet on the bottom part of the storage container. In figure 4A the storage container is in a side view, in figure 4B the storage container is in a frontal view, and in figure 4C a bottom view is shown.

Corresponding reference characters indicate corresponding parts throughout the several views. Although the drawings represent an embodiment of the present invention, the drawings are not necessarily to scale, and certain features may be exaggerated or omitted in some of the drawings in order to better illustrate and explain the present invention.

### DETAILED DESCRIPTION OF THE INVENTION

Referring to FIG. 1, there is shown an exemplary embodiment of a storage container for an electronic injection device of the present invention. When in a closed arrangement shown in FIG. 1A, the storage container protectively houses the electronic injection device previously received therein. The storage container is shown having a bottom part (1) and a top part or lid (2). In the closed arrangement the container is hermetically sealed in part due to a seal (7), as shown in FIG 1B. Such seal (7) is located between the bottom (1) and top (2) parts of the storage container, in circumference of that portion of the bottom part (1) which is in contact with a rim (8) of the top part (2). Alternatively, the rim (8) of the top part (1) may also contain along its edge a seal. The seal (7) may be of any malleable material commonly used as a seal between two or more moveable parts. The seal material need to be able to provide a sufficient barrier between the interior space and the outside environment at a range of temperatures, preferably from 0°C to 60°C, more preferable from 0°C to 45°C. Examples of such seal material include silicon and rubber. Such seal (7) may be a tubular or squared, either of which may be solid or hollow, material forming a ring in circumference with the edge of either the bottom part (1) or top part (2) or both.

In order to more securely close the storage container one or more counterpart latches (9a, 9b) may be provided as shown in FIG. 1B. Each one of the counterpart latches (9a, 9b) being located on the edge of either the bottom part (1) or the top part (2) opposite the other of the counterpart latch (9a, 9b). The storage container may contain one or more of such counterpart lathes. The latching mechanism may in addition prevent inadvertent opening of the case.

The top part or lid (2) may be removed away from the bottom part (1) so as to open the storage container. Alternatively, the top part or lid (2) may be hingedly connected to the bottom part (1) of the storage container with one or more hinges (6). Opening of the storage container can thus be accomplished in several manners such as for example the total removal of the top part or lid (2) from the bottom part (1), or by moving the top part or lid (2) by using one or more hinges (6). However, otherwise designed storage containers that may be opened and closed may naturally be employed within the scope of the invention.

The top part or lid (2) may be a solid lid or alternatively includes an outer shell and an interior space. Such interior space may be contoured or molded to match and fit the exposed portions of the compartment(s) (5) and the electronic injection device (3), which are exposed from the bottom part after being received therein. However, the top part or lid (2) should not impede the temperature equilibration inside a closed storage container with the outside environment. As such the top part or lid (2) may be constructed of any rigid non-permeable material such as plastic, preferably a thermoplastic material of a limited thickness. Examples of such thermoplastic material includes acrylonitrile butadiene styrene (ABS), polypropylene (PP), polycarbonate (PC), polyethylene (PE), low density polyethylene (LDPE), high density polyethylene (HDPE), polyvinylchloride (PVC), polystyrene (PS) and polyethylene terephthalate (PET), preferable the thermoplastic material is polypropylene

(PP). Preferably the thickness of this material for the top part or lid (2) is from 0.5 mm to 3.0 mm, more preferably from 1.0 mm to 2.0 mm.

The interior space (10) of bottom part (1), as shown in FIG. 2, comprises a compartment (5) for containing a moisture absorbing material. In addition, this interior space may further contain molded or contoured surface(s) (12), such as forming a recess, for providing a close fit for the electronic injection device (3) when housed in the bottom part (1) of the storage container. Such molded or contoured surface(s) (12) should not impede the heat exchange and equilibration of the temperature of the electronic injection device (3) when housed in a close storage container with the outside temperature. Preferably any such surface should have a thickness of less than 3.5mm, less than 2.5 mm, or from 0.5 mm to 2.0 mm. The compartment (5) for holding a moisture absorbing material, desiccant, in a preferred embodiment of the present invention is also contoured or molded such that it will closely fit in the interior surface (10). Such contoured or molded compartment (5) may also further secure the electronic injection device (3) in place when it is housed in the storage container. The compartment (5) for holding the moisture absorbing material or desiccant further comprises at least one opening for providing air flow communication between the interior space (10), also holding the electronic injection device (3), and the moisture absorbent or desiccant, such that moisture in that internal volume can be drawn off. This compartment (5) for containing the moisture absorbent or desiccant may be removed from the interior space (10) of the bottom part (1) and can be secured in place such as via snap fits, press fits, or another suitable manner. The removal of this compartment (5) from the storage container allows for the replenishment or exchange to fresh moisture absorbent or desiccant for the continued use of the storage container after the moisture absorbent capacity of the desiccant has been exhausted, i.e. the desiccant is saturated with moisture and no longer operates efficiently as desiccant.

The storage container thus includes a moisture absorbing material or desiccant to reduce the likelihood that the intended contents of the container, for example the electronic injection device (3), will be damaged from moisture when housed therein. The material can be in the form of a contained desiccant, such as a desiccant pack, held in the shown embodiment by the compartment (5) inside the interior space of the bottom part (1) of the storage container. Although shown being located only in that compartment, in alternative embodiments, the desiccant can be provided in one or more such compartments which may also be located around the electronic injection device (3) or in the top part or lid (2) of the storage container. A suitable desiccant can be silica gel, molecular sieve, or clay.

The electronic injection (3) device may be any electronic injection device for delivering medication to a patient such as for example the device described in WO2005/077441. The electronic injection device (3), when not being stored within storage container, is operable to deliver medication into a user in a known fashion. The electronic injection device uses electronic circuitry, such as circuitry used to accurately indicate the dose of medication being injected into the user, or to record data about the injection, or to actually drive the injection. The storage container advantageously provides for storing such devices having electronic components which are potentially adversely impacted by moisture within the device.

Considering that certain electronic injection devices are stored containing a multi-dose medication cartridge inside the storage container of the present invention is particularly beneficial for such electronic injection device. However, the storage container should not impede the convenience of the user/patient in use. Accordingly, equilibration of the temperature of the electronic injection device (3) housed in the closed storage container when removed from a refrigerator to an ambient environment should not be impeded. In fact, the period for equilibrating such device temperature to the ambient temperature should be less than about 60 minutes, preferably less than about 30 minutes, more preferably less than about 20 minutes. Preferably the time period for equilibrating the electronic injection device after removing the storage container from the refrigerator (cooled storage conditions) is about 15 minutes or less.

In order to achieve a sufficiently rapid heat exchange between the interior space (10) of the closed storage container and the outside environment after removal of the storage container from the cooled storage conditions the outer surface (4) of the bottom part (1) of the storage container is of a thermoplastic material which allows for such heat exchange. In addition, the outer surface (4) of the bottom part (1) should be of limited thickness. Preferably the thickness is from 0.5 mm to 3.5 mm, more preferably from about 1.0 mm to about 2.5 mm, even more preferably from about 1.5 mm to about 2.5 mm. The thermoplastic material for the outer surface (4) of the bottom part (1) of the storage container can be any non-permeable thermoplastic material preferably selected from for example acrylonitrile butadiene styrene (ABS), polypropylene (PP), polycarbonate (PC), polyethylene (PE), low density polyethylene (LDPE), high density polyethylene (HDPE), polyvinylchloride (PVC), polystyrene (PS) and polyethylene terephthalate (PET). Preferable the thermoplastic material is polypropylene (PP). Alternatively in order to limit the thickness of the material of the outer surface (4) this surface can be formed from a hollow shell material with a surface facing outward and an internal liner (13) as shown in FIG. 3. The space in between the surface facing outward and the internal liner can be for example open to the air. As shown in FIG. 4, in one embodiment the storage container comprises one or more feet (14) on which the bottom part rests when used to store an electronic injection device. Such one or more feet (14) can allow ventilation of the air underneath the bottom part of the storage container and/or air exchange of the air between the liner (13) and the outer surface (4). Airflow or ventilation of the air in close contact with that part of the bottom compartment which houses the electronic injection device (3) can improve the heat exchange between the ambient environment and the interior space (10) of the storage container when removed from for example a refrigerated environment.

In an alternative embodiment the outer surface (4) on at least part of its surface contains a roughed profile (11). Such roughed profile (11) improves the grip for the user/patient on the storage container. This is particularly beneficial for patients who suffer from an illness or disease which compromises their dexterity abilities. This may also be true for older patients or very young patients. Additionally, condensation on the outside of the storage container may impact the ease of gripping the container and such roughed profile (11) on the outer surface (4) of the storage container improves such ease of gripping the container.

Optionally, the storage container of the present invention is balanced such that the storage container is stable in upright position when positioned on a smooth surface or grill type surface in for example a refrigerator. Such balance of the storage container is independent on whether the compartment (5) is filled with a moisture absorbent material (desiccant) or not.

## Claims

1. A storage container for an electronic injection device comprising;
a bottom part (1) having an outer surface (4) and defining an interior space (10) for holding the electronic injection device;
a movable top part (2) for closing the interior space of the bottom part forming when closed a hermetically sealed interior compartment; and
a desiccant in air flow communication with the interior space, the desiccant being in a separate compartment (5) in the interior space of the bottom part of the storage container, the separate compartment comprising at least one opening for providing air flow communication with the interior space,
wherein the compartment containing the desiccant is replaceable from the interior space of the storage container, and
wherein the outer surface comprises a hollow shell material with a surface facing outward and an internal liner (13).

2. The storage container according to claim 1, wherein the bottom part of the storage container comprises feet on which the bottom part rests.

3. The storage container according to any of the preceding claims, wherein the outer surface comprises a non-permeable material.

4. The storage container according to any of the preceding claims, wherein the outer surface comprises a thermoplastic material and has a thickness of less than 3.5 mm.

5. The storage container according to claim 4, wherein the thermoplastic material is selected from acrylonitrile butadiene styrene (ABS), polypropylene (PP), polycarbonate (PC), polyethylene (PE), low density polyethylene (LDPE), high density polyethylene (HDPE), polyvinylchloride (PVC), polystyrene (PS) and polyethylene terephthalate (PET).

6. The storage container according to claim 5, wherein the thermoplastic material is polypropylene.

7. The storage container according to any of claims 4 to 6, wherein the outer surface has a thickness of 0.5 to 3.5 mm.

8. The storage container according to claim 7, wherein the outer surface has a thickness of 1.0 mm to 2.5 mm.

9. The storage container according to any of the preceding claims, wherein the storage container is stable in upright position when positioned on a smooth surface or grill type surface in a refrigerator.

10. The storage container according to any of the preceding claims, wherein either the top part is removable from the bottom part or the bottom part and top part are hingedly connected to be pivotably moveable between a closed and open position.

11. The storage container according to any of the preceding claims, wherein the interior space comprises a recess for providing a close fit for the electronic injection device.

12. The storage container according to any of the preceding claims, wherein at least a portion of the outer surface comprises a roughed profile (11).

13. The storage container according to claim 1, wherein the space in between the surface facing outward and the internal liner is open to the air.

## Patentansprüche

1. Aufbewahrungsbehälter für eine elektronische Injektionsvorrichtung, umfassend;
ein Bodenteil (1), das eine Außenfläche (4) aufweist und einen Innenraum (10) zum Halten der elektronischen Injektionsvorrichtung definiert;
ein bewegliches Oberteil (2) zum Verschließen des Innenraums des Bodenteils, das im geschlossenen Zustand einen hermetisch abgedichteten Innenteilraum bildet; und
ein Trockenmittel in Luftstromverbindung mit dem Innenraum, wobei sich das Trockenmittel in einem separaten Teilraum (5) in dem Innenraum des Bodenteils des Aufbewahrungsbehälters befindet, wobei der separate Teilraum mindestens eine Öffnung umfasst, um eine Luftstromverbindung mit dem Innenraum herzustellen,
wobei der Teilraum, der das Trockenmittel enthält, von dem Innenraum des Aufbewahrungsbehälters aus austauschbar ist, und
wobei die Außenfläche ein hohles Mantelmaterial mit einer nach außen gerichteten Oberfläche und eine Innenauskleidung (13) umfasst.

2. Aufbewahrungsbehälter nach Anspruch 1, wobei das Bodenteil des Lagerbehälters Füße umfasst, auf denen das Bodenteil ruht.

3. Aufbewahrungsbehälter nach einem der vorhergehenden Ansprüche, wobei die Außenfläche ein nicht durchlässiges Material umfasst.

4. Aufbewahrungsbehälter nach einem der vorangehenden Ansprüche, wobei die Außenfläche ein thermoplastisches Material umfasst und eine Dicke von weniger als 3,5 mm aufweist.

5. Aufbewahrungsbehälter nach Anspruch 4, wobei das thermoplastische Material ausgewählt ist aus Acrylnitril-Butadien-Styrol (ABS), Polypropylen (PP), Polycarbonat (PC), Polyethylen (PE), Polyethylen niedriger Dichte (LDPE), Polyethylen hoher Dichte (HDPE), Polyvinylchlorid (PVC), Polystyrol (PS) und Polyethylenterephthalat (PET).

6. Aufbewahrungsbehälter nach Anspruch 5, wobei das thermoplastische Material Polypropylen ist.

7. Aufbewahrungsbehälter nach einem der Ansprüche 4 bis 6, wobei die Außenfläche eine Dicke von 0,5 bis 3,5 mm aufweist.

8. Aufbewahrungsbehälter nach Anspruch 7, wobei die Außenfläche eine Dicke von 1,0 mm bis 2,5 mm aufweist.

9. Aufbewahrungsbehälter nach einem der vorhergehenden Ansprüche, wobei der Aufbewahrungsbehälter in aufrechter Position stabil ist, wenn er auf einer glatten Oberfläche oder einer grillartigen Oberfläche in einem Kühlschrank positioniert ist.

10. Aufbewahrungsbehälter nach einem der vorhergehenden Ansprüche, wobei entweder das Oberteil von dem Bodenteil abnehmbar ist oder das Bodenteil und das Oberteil klappbar verbunden sind, um zwischen einer geschlossenen und einer offenen Position schwenkbar zu sein.

11. Aufbewahrungsbehälter nach einem der vorhergehenden Ansprüche, wobei der Innenraum eine Aussparung umfasst, um eine Feinpassung für die elektronische Injektionseinrichtung bereitzustellen.

12. Aufbewahrungsbehälter nach einem der vorhergehenden Ansprüche, wobei zumindest ein Abschnitt der Außenfläche ein aufgerautes Profil (11) umfasst.

13. Aufbewahrungsbehälter nach Anspruch 1, wobei der Raum zwischen der nach außen gerichteten Oberfläche und der Innenauskleidung zur Luft hin offen ist.

## Revendications

1. Conteneur de stockage pour un dispositif d'injection électronique comprenant :
une partie inférieure (1) comportant une surface extérieure (4) et définissant un espace intérieur (10) pour contenir le dispositif d'injection électronique ;
une partie supérieure mobile (2) pour fermer l'espace intérieur de la partie inférieure formant quand elle est fermée un compartiment intérieur hermétiquement scellé ; et
un dessiccant en communication par écoulement d'air avec l'espace intérieur, le dessiccant étant dans un compartiment séparé (5) dans l'espace intérieur de la partie inférieure du conteneur de stockage, le compartiment séparé comprenant au moins une ouverture pour permettre la communication par écoulement d'air avec l'espace intérieur,
dans lequel le compartiment contenant le dessiccant peut être remplacé depuis l'espace intérieur du conteneur de stockage, et
dans lequel la surface extérieure comprend un matériau de coque creuse avec une surface faisant face vers l'extérieur et une chemise intérieure (13).

2. Conteneur de stockage selon la revendication 1, dans lequel la partie inférieure du conteneur de stockage comprend des pieds sur lesquels la partie inférieure repose.

3. Conteneur de stockage selon l'une quelconque des revendications précédentes, dans lequel la surface extérieure comprend un matériau non perméable.

4. Conteneur de stockage selon l'une quelconque des revendications précédentes, dans lequel la surface extérieure comprend un matériau thermoplastique et a une épaisseur de moins de 3,5 mm.

5. Conteneur de stockage selon la revendication 4, dans lequel le matériau thermoplastique est sélectionné parmi l'acrylonitrile butadiène styrène (ABS), le polypropylène (PP), le polycarbonate (PC), le polyéhylène (PE), le polyéthylène basse densité (LDPE), le polyéthylène haute densité (HDPE), le polychlorure de vinyle (PVC), le polystyrène (PS) et le polytéréphtalate d'éthylène (PET).

6. Conteneur de stockage selon la revendication 5, dans lequel le matériau thermoplastique est du polypropylène.

7. Conteneur de stockage selon l'une quelconque des revendications 4 à 6, dans lequel la surface extérieure a une épaisseur de 0,5 à 3,5 mm.

8. Conteneur de stockage selon la revendication 7, dans lequel la surface extérieure a une épaisseur de 1,0 à 2,5 mm.

9. Conteneur de stockage selon l'une quelconque des revendications précédentes, dans lequel le conteneur de stockage est stable en position verticale quand il est positionné sur une surface lisse ou une surface du type grille dans un réfrigérateur.

10. Conteneur de stockage selon l'une quelconque des revendications précédentes, dans lequel soit la partie supérieure est amovible de la partie inférieure soit la partie inférieure et la partie supérieure sont connectée de façon articulée pour être mobiles en pivotant entre une position ouverte et fermée.

11. Conteneur de stockage selon l'une quelconque des revendications précédentes, dans lequel l'espace intérieur comprend un évidement pour permettre un ajustement étroit pour le dispositif d'injection électronique.

12. Conteneur de stockage selon l'une quelconque des revendications précédentes, dans lequel au moins une partie de la surface extérieure comprend un profil rugueux (11).

13. Conteneur de stockage selon la revendication 1, dans lequel l'espace entre la surface faisant face à l'extérieur et la chemise intérieure est ouvert à l'air.
